# EUROPÄISCHE PATENTSCHRIFT

(11) **EP 2 082 219 B1**
(45) Veröffentlichungstag und Bekanntmachung des Hinweises auf die Patenterteilung: **09.02.2011**
(21) Anmeldenummer: 07856004.2
(22) Anmeldetag: 12.11.2007
(51) Int. Cl.: G01N 27/12, G01N 33/22

(54) **VERWENDUNG EINES SUBSTRATS ZUR DETEKTION CHEMISCHER DOTIERSTOFFE**
USE OF A SUBSTRATE FOR THE DETECTION OF CHEMICAL DOPANTS
UTILISATION D'UN SUBSTRAT POUR DÉTECTER DES AGENTS DOPANTS

(30) Priorität: 14.11.2006 DE 102006053890
(43) Veröffentlichungstag der Anmeldung: 29.07.2009
(73) Patentinhaber: EADS Deutschland GmbH, 85521 Ottobrunn (DE)
(72) Erfinder: EICKHOFF, Martin, 35398 Giessen-Allendorf (DE); HELWIG, Andreas, 80469 München (DE); GARRIDO, José Antonio, 85435 Erding (DE); MÜLLER, Gerhard, 85567 Grafing (DE)
(74) Vertreter: Meel, Thomas
(86) Internationale Anmeldenummer: PCT/DE2007/002040
(87) Internationale Veröffentlichungsnummer: WO 2008/058518

(56) Entgegenhaltungen:
- HELWIG A ET AL: "Gas sensing interactions at hydrogenated diamond surfaces" IEEE SENSORS JOURNAL, Bd. 7, Nr. 9, September 2007 (2007-09), Seiten 1349-1353, XP011189507 ISSN: 1530-437X
- REZEK ET AL: "Intrinsic hydrogen-terminated diamond as ion-sensitive field effect transistor" SENSORS AND ACTUATORS B, Bd. 122, Nr. 2, 17. August 2006 (2006-08-17), Seiten 596-599, XP005924563 ISSN: 0925-4005
- GI R S ET AL: "POSSIBILITY OF REALIZING A GAS SENSOR USING SURFACE CONDUCTIVE LAYER ON DIAMOND FILMS" JAPANESE JOURNAL OF APPLIED PHYSICS, JAPAN SOCIETY OF APPLIED PHYSICS, TOKYO, JP, Bd. 36, Nr. 4A, April 1997 (1997-04), Seiten 2057-2060, XP000732319 ISSN: 0021-4922
- TANIELIAN M ET AL: "Effect of adsorbates and insulating layers on the conductance of plasma deposited a-Si:H" JOURNAL OF NON-CRYSTALLINE SOLIDS, Bd. 35-36, Januar 1980 (1980-01), Seiten 575-580, XP002477733 ISSN: 0022-3093 in der Anmeldung erwähnt

## Beschreibung

Die Erfindung betrifft eine besondere Verwendung eines Substrats zur Detektion chemischer Gefahrstoffe.

Dazu gehören Phosphin (PH₃), Arsin (AsH₃), Diboran (B₂H₆). Diese bilden eine Gruppe hochgiftiger Dotierstoffen, die routinemäßig in der Halbleiterindustrie eingesetzt werden.

Es gibt daher ein starkes Bedürfnis, das Vorhandensein solch hochgefährlicher Stoffe in verschiedenen Umgebungen, insbesondere in der Luft, schnell detektieren zu können.

Zur Detektion von oben genannten Gasen und Dämpfen gibt es mehrere Sensorsysteme auf dem Markt. Diese sind zum einen Teil teure und hochkomplexe analytische Instrumente, die nur von geschultem Personal zu bedienen sind und deren Resultate nur von solchem Personal sicher interpretiert werden können. Beispiele sind Gaschromatographen, Massen- und Ionen-Mobilitätsspektrometer. Preiswerte marktgängige Sensoren wie z.B. Dickschichtmetalloxidsensoren können solche gefährlichen Gase zwar in niedrigen Konzentrationen nachweisen. Ein gravierender Nachteil solch preiswerter Sensoren ist jedoch die Tatsache, dass diese ebenfalls auf eine große Anzahl von Störgasen reagieren, die in relativ hohen und stark variablen Konzentrationen ebenfalls in der Luft vorliegen können. Der Grund für die mangelnde Selektivität ist u.a. auch in der hohen Betriebstemperatur von ca. 400°C begründet, bei der die meisten nachzuweisenden Moleküle an der Sensoroberfläche verbrannt und dadurch erst elektrisch nachgewiesen werden. Zur genaueren Identifizierung müssen deshalb mehrere Sensoren mit verschiedenen Querempfindlichkeiten in einem Sensorarray parallel betrieben werden (sog. elektronische Nasen). Eine eindeutige Detektion chemischer Kampfstoffe mit Hilfe elektronischer Nasen war aus den o.g. Gründen jedoch bisher in den meisten Fällen noch nicht mit der notwendigen Sicherheit möglich.

Aufgabe der Erfindung ist es daher, eine schnelle und unkomplizierte Detektion zumindest einiger der vorgenannten chemischen Dotierstoffe in der Luft bei Raumtemperatur zu ermöglichen, ohne auf die ebenfalls in verschiedenen industriellen Umgebungen und/oder in der Umwelt vorkommenden Störgase zu reagieren.

Diese Aufgabe wird gelöst durch die besondere Verwendung eines an sich bekannten Substrats gemäß Patentanspruch 1.

Vorteilhafte Ausgestaltungen der Erfindung sind Gegenstand der Unteransprüche.

Durch die erfindungsgemäße Verwendung des Substrats lassen sich chemische Dotierstoffe in Gasen und insbesondere in der Luft detektieren, ohne dass eine Störung der Detektion durch andere vorhandene Stoffe erfolgt. Das heißt, eine Überdeckung des von den hochgiftigen bzw. tödlich wirkenden Gefahr- oder Kampfstoffen verursachten Messsignals durch Signale, die von störenden Hintergrundstoffen verursacht werden, wird vermieden. Solche Störgase sind beispielsweise CO, 03, Erdgas (insbesondere CH4) oder auch alkoholische Dämpfe.

Vorteilhaft ist die Dichte der Wasserstoffbelegung an der Oberfläche durch partielle Oxidation reduziert. Dadurch wird eine Empfindlichkeitssteigerung erreicht.

Erfindungsgemäß eignet sich das Substrat zur Detektion von hochgiftigen Dotierstoffen, die chemisch zu den Gruppen der III-H-, V-H- Verbindungen gehören.

Die Erfindung basiert auf der Überlegung, dass die meisten dieser Stoffe doppelt besetzte Elektronenorbitale aufweisen, die an keiner kovalenten Bindung innerhalb des nachzuweisenden Moleküls teilnehmen. Die hydrogenisierte bzw. wasserstoffterminierte Oberfläche des Substrats bietet eine Andockmöglichkeit über Wasserstoffbrückenverbindungen. Die nachfolgend beschriebenen Messergebnisse belegen, dass über solche Physisorptionsbindungen mit ausreichend hoher Wahrscheinlichkeit elektrische Ladungen zwischen den Kampfstoffverbindungen und dem Sensorsubstrat übertragen werden können. Solche Ladungsüberträge führen zu einer Veränderung der elektrischen Leitfähigkeit an der Substratoberfläche und damit zu einem nachweisbaren Sensorsignal.

Vorzugsweise werden an der Wasserstoff(H)-terminierten Oberfläche mittels einer Messeinrichtung Verschiebungen von Oberflächenladungen gemessen.

Insbesondere bildet eine auf das Substrat aufgebrachte Schicht von Wasserstoffatomen die Wasserstoff(H)-terminierte Oberfläche. Die Schicht von Wasserstoffatomen ist zum Beispiel eine monoatomare Schicht.

Das Substrat kann zum Beispiel aus Diamant, hydrogenisiertem amorphem Silizium (a-Si:H), Sliziumcarbid, einem Gruppe III-Nitrid oder einem Metalloxid gefertigt sein. Konkrete Beispiele aus den letzteren beiden Gruppen sind GaN oder Zinnoxid oder auch Zinkoxid.

Der elektrische Widerstand wird bevorzugt an der Wasserstoff(H)-terminierten Oberfläche mittels einer Messeinrichtung gemessen. Die Messung zur Detektion der genannten Gase oder Dämpfe erfolgt vorzugsweise bei einer Temperatur unterhalb von 100°C, insbesondere auch bei Raumtemperatur.

Die sensitive Wasserstoff(H)-terminierte Substratoberfläche wird vorzugsweise durch Spülung mit einem Fluid, das die zu detektierenden Stoffe nicht enthält, insbesondere Luft, gereinigt. Die Reinigung erfolgt bevorzugt unter Zugabe eines oxidierenden Fluids, wie insbesondere Ozon.

Erfindungsgemäß wird das Substrat zur Detektion eines oder mehrerer der folgenden Gase verwendet: B₂H₆, PH₃, AsH₃.

Durch die erfindungsgemäße Verwendung lässt sich ein Detektor bilden, der ein Substrat, das eine dem Gas auszusetzende Wasserstoff(H)-terminierte Substratoberfläche aufweist, sowie eine Messeinrichtung zum Messen von Verschiebungen von Oberflächenladungen an der Wasserstoff(H)-terminierten Substratoberfläche umfasst, wobei das Substrat aus einem Nichtleitermaterial mit einer Oberflächenleitfähigkeit oder aus einem Halbleitermaterial gefertigt ist.

Vorzugsweise ist zum Bilden der Wasserstoff(H)-terminierten Substratoberfläche auf das Substrat eine Schicht von Wasserstoffatomen aufgebracht.

Weiter kann eine Ausleseeinheit kann z.B. in Form eines Transistors, verwendet werden. Die Ausleseeinheit und die empfindliche Schicht, z.B. a-Si:H oder H-terminierter Diamant, können aus unterschiedlichen Materialien gefertigt sein, d.h. die sensitive Schicht wird zunächst auf die Ausleseeinheit aufgebracht.

Erfindungsgemäß wird demnach zum Beispiel eine Halbleiteroberfläche mit einer Schicht von atomarem Wasserstoff terminiert. Eine solche hydrogenisierte Halbleiteroberfläche bildet die sensitive Schicht, an die die nachzuweisenden Kampfstof fe andocken. Die H-Terminierung ermöglicht z.B. eine Anlagerung eines zu detektierenden Gruppe V-H - Moleküls, und die nachfolgende Bildung eines elektrisch geladene Komplexes aus diesem Molekül mit dem Oberflächen-gebundenen Wasserstoff bei Raumtemperatur. Störende Gase reagieren erst bei höheren Temperaturen an der Oberfläche und stellen somit keine Gefahr der Verfälschung dar.

Ein sich durch eine erfindungsgemäße Verwendung ergebender Detektor hat einen geringeren Leistungsverbrauch als bestehende Konkurrenzprodukte, da keine Heizleistung für die sensitive Schicht benötigt wird.

Weiter ergibt sich ein unkomplizierter Aufbau und ein geringer Leistungsverbrauch, da nur ein einzelner Sensor ausreicht und keine weiteren Sensoren zur Ausfilterung von Störgasen benötigt werden.

Ein durch die erfindungsgemäße Verwendung geschaffener Sensor zeichnet sich insbesondere dadurch aus, dass er keine Querempfindlichkeit auf Umwelteinflüsse und störende Gase in der industriellen Fertigung hat.

Nach einer Messung kann die Sensoroberfläche durch eine hohe Konzentration von Ozon gereinigt werden.

Der erfindungsgemäß verwendete Effekt einer Änderung der Oberflächenleitfähigkeit von hydrogenisierten Halbleiteroberflächen durch verschiedene Gruppe-V-H-Verbindungen ist in der Literatur schon länger bekannt, zumeist bei hydrogenisiertem amorphen Silizium. Dieses Material wird verstärkt in der Photovoltaik eingesetzt.

So beschreibt der Artikel "Effect of Adsorbates and Insulating Layers on the Conductance of Plasma Deposited a-Si:H*" von M. Tanielian et al. in Journal of Non-Crystalline Solids 35 & 36 (1980) 575-580, dass verschiedene adsorbierte Gase wie H₂O und besonders NH₃ einen starken Effekt auf die Leitfähigkeit und die Fotoleitfähigkeit von Hochwiderstandsschichten aus amorphen hydrogenisierten Silizium haben. Es wird beschrieben, dass NH₃ bei hydrogenisiertem amorphen Silizium als Oberflächendonator wirkt und dadurch die Oberflächenleitfähigkeit verändert. Dagegen wirkt Selen als Akzeptor und verändert ebenfalls die Oberflächenleitfähigkeit. Es wird weiter beschrieben, dass durch diese Stoffe erzeugte Oberflächenladungsveränderungen durch Erhitzen im Vakuum rückgängig gemacht werden können.

Frank J. Kampas beschreibt in "Chemical Reactions in Plasma Deposition", Kapitel 8 aus "Semiconductors and Semimetals", Vol. 21A, Academic Press, Inc., 1984, welche chemischen Prozesse beim Wachstum von hydrogenisierten amorphen Silizium schichten ablaufen und wie sich augrund des speziellen Wachstumsprozesses sich eine natürlicherweise hydrogenisierte Oberfläche ausbildet. G. Müller et al. (Journal of Non-Crystalline Solids 59 & 60 (1983), S. 469-472) präsentieren in dem Artikel "Hydrogen Incorporation, Doping and Thickness Dependent Conductivity in Glow Discharge Deposited a-Si:H Films" Wasserstofftiefenprofile durch a-Si:H-Schichten verschiedener Dicke und zeigen explizit, dass eine hohe Oberflächedichte von Wasserstoffatomen vorliegt.

R. Sung Gi et. al. beschreiben in dem Artikel "Hall Effect Measurements of Surface Conductive Layer of undoped Diamond Films in NO2 and NH3 Atmospheres", Jpn. J. Appl. Phys. Vol. 38 (1999), S. 3492 - 3496, NO₂ und NH₃ induzierte Änderungen der elektrischen Oberflächenleitfähigkeit in Diamantproben mit hydrogenisierten Oberflächen.

Die Erfindung nutzt nun den Effekt der Verschiebung von Oberflächenladungen über die H - terminierte Substrat- bzw. Halbleiteroberfläche hinweg erstmals zur gezielten Detektion von gasförmigen oder dampfförmigen chemischen Kampfstoffen.

Ausführungsbeispiele der Erfindung werden nachfolgend anhand der hier beigefügten Figuren näher erläutert. Darin zeigt:
- **Fig. 1**: einen Graph, der den Widerstand eines hydrogenisierten Diamanten bei Raumtemperatur unter unterschiedlichen Atmosphären verdeutlicht;
- **Fig. 2**: einen Graph, der den NH3 Response bei verschiedenen Temperaturen zeigt;
- **Fig. 3**: einen Graph, der zeigt wie durch Beleuchtung mit ultraviolettem Licht die Ansprech- und Abklinggeschwindigkeit gesteigert werden kann.
- **Fig. 4**: einen Graph, der die Reinigung einer auf verschiedene Gase sensitiven Schicht durch Ozon verdeutlicht;
- **Fig. 5**: einen Graph, der die Empfindlichkeit der hydrogenisierten Oberfläche auf NO₂ zeigt.
- **Fig. 6**: einen Graph, der die Empfindlichkeitsteigerung verdeutlicht, die durch "Verdünnung" der hydrogenisierten Oberfläche durch partielle Oxidation verdeutlicht.

Als vorbereitende Untersuchung zur Herstellung eines Detektors in Form eines Gassensors wurde auf einem Diamantsubstrat eine H-terminierte Oberfläche hergestellt. Die auf dem Diamantsubstrat befindliche monoatomare Schicht wurde bei mehreren Arbeitstemperaturen auf ihre Empfindlichkeit gegenüber verschiedener Gase untersucht. Die Schicht hat sich als selektiv gegenüber Ammoniak und NO₂ bei Raumtemperatur erwiesen. Es kann noch eine Erhöhung der Response oder Sensitivität durch teilweise Ersetzung der H-Terminierung durch O-Terminierung erfolgen.

Ein so hergestellter Gassensor kann als Schwellwertsensor für die Fertigung in der chemischen Industrie und in der Halbleiterindustrie eingesetzt werden.

Als Grundlage zur Schaffung eines Detektors ist stellvertretend für Diboran (B₂H₆), Phosphin (PH₃) und Arsin (AsH₃) das demgegenüber relativ ungiftige Ammoniak (NH₃) näher untersucht worden.

Als mögliche Substrate, auf welche eine hydrogenisierte, sensitive Schicht aufgebracht werden kann, eignen sich besonders Halbleitermaterialien, wie z. B. Diamant, Siliziumcarbid, alle Gruppe III-Nitride (z. B. GaN) und Metalloxide (z. B. Zinnoxid) oder auch Silizium.

Eine hydrogenisierte Oberfläche ist eine Oberfläche, die durch Wasserstoffatome terminiert ist. Diese Terminierung kann in unterschiedlichen Verfahren, z. B. in einem Wasserstoffplasma oder in einer "Hot-wire" Hydrogenisierung aufgebracht werden. Ein hydrogenisiertes Layer ist eine monoatomare Schicht bestehend aus Wasserstoffatomen, die kovalent an die Gitterbestandteile des Substrats gebunden ist. Die Oberfläche ist somit mit einer Schicht von Wasserstoffatomen überzogen. Eines von mehreren für die Wasserstoffterminierung geeigneten Verfahren ist eine Wasserstoffglimmentladung. Als weitere Verfahren kommen die Silanisierung und chemische Funktionalisierung von Oberflächen mit organischen Molekülen in Betracht.

Diese an der Oberfläche liegenden H-Atome begünstigen schon bei niederen Temperaturen (z. B. Raumtemperatur - RT) die Adsorption in diesem Fall von NH₃-Molekülen und bilden mit diesen einen NH₃ - H - Komplex. Es erfolgt eine Verschiebung der Oberflächenladung, die mit Hilfe geeigneter Messtechnik elektrisch ausgelesen werden kann. Hierzu wird eine Messeinrichtung zum Messen des Oberflächenwiderstandes eingesetzt. Messeinrichtungen zum Messen des ohmschen Widerstandes sind gut bekannt und müssen hier nicht näher erläutert werden.

Andere Gase, die täglich in der Umwelt vorkommen (z. B. Ethanol, Kohlenwasserstoffe, CO, Wasserstoff), benötigen höhere Energien, um sich an einer hydrogenisierten Oberfäche anzulagern und chemisch zu reagieren. Somit wird bei niederen Temperaturen, hier kleiner als 100°C, als reduzierendes Gas nur das NH₃ und chemisch verwandte Stoffe wie PH3 und AsH3 detektiert. Genauer untersucht wurde die Detektion von NH₃ mittels eines hydrogenisierten Diamanten. Die Messergebnisse werden im Folgenden anhand der beigefügten Figuren näher erläutert.

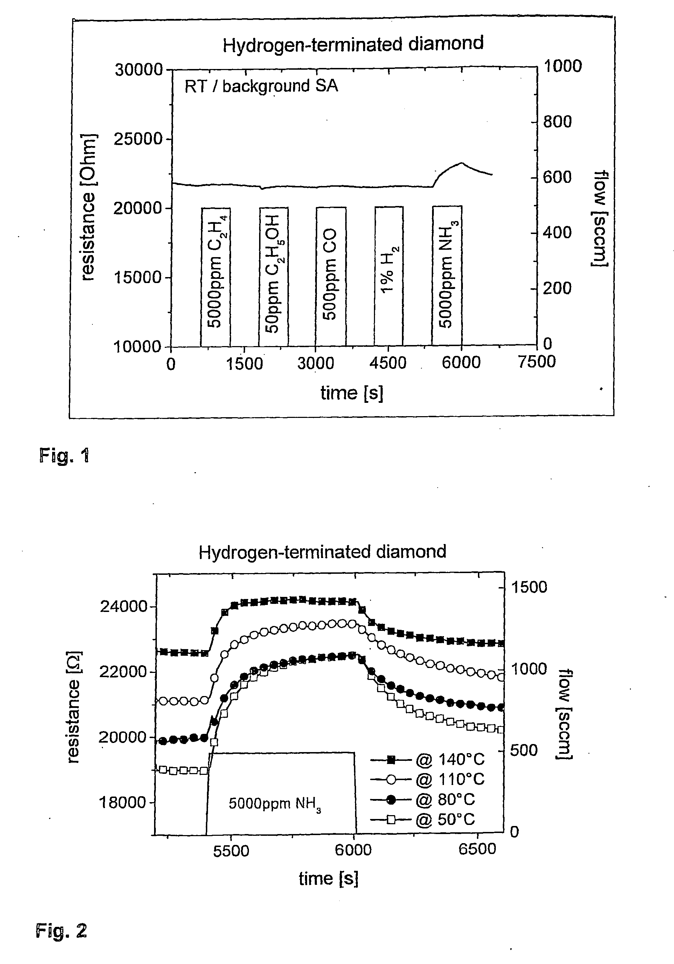

Fig. 1 zeigt das Widerstandsverhalten eines hydrogenisierten Diamanten unter unterschiedlichen Gasatmosphären bei Raumtemperatur RT. Als Hintergrund-Atmosphäre wurde synthetische Luft SL eingesetzt. Bei diesem Versuch wurden zeitlich versetzt Gaspulse von unterschiedlichen Gasen in ungewöhnlich hoher Konzentration eingeleitet. Nach einem Gaspuls erfolgte dann jeweils ein Spülpuls mit synthetischer Luft (SL), die aus 20 % Sauerstoff und 80 % Stickstoff gebildet wurde. Wie aus dem Graph von Fig. 1 ersichtlich, wurden zunächst 5.000 ppm (parts per million) Ethen, dann 50 ppm Ethanol, dann 500 ppm Kohlenstoffmonooxid, dann 1 % Wasserstoff und schließlich 5.000 ppm Ammoniak eingeleitet. Dabei wurde der Oberflächenwiderstand permanent gemessen. Ersichtlich gab es nur bei der Einleitung des Ammoniaks eine Änderung des Widerstandes.

Fig. 2 zeigt die Wiederholung des Experimentes gemäß Fig. 1, allerdings bei sukzessiv höheren Arbeitstemperaturen des Diamanten. Erneut wurde nur bei Beimischung von NH₃ eine Erhöhung des Bauteilwiderstandes beobachtet. Betrachtet man den Zeitbereich, in dem NH3 beaufschlagt wurde genauer, so erkennt man eine Verschiebung der Sensorbasislinie hin zu einem geringeren Grundwiderstand in synthetischer Luft bedingt durch die höhere Temperatur. Mit wachsender Arbeitstemperatur werden jedoch deutlich kürzere Reaktionszeiten für die Adsorption und Desorption des NH3 beobachtet.

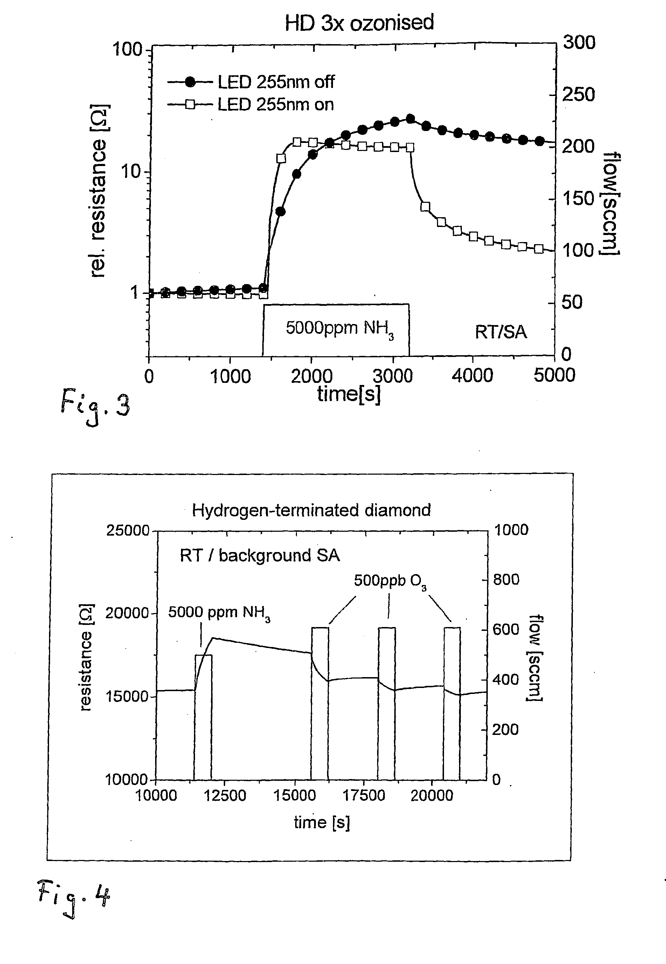

Fig. 3 zeigt die beschleunigende Wirkung von ultraviolettem (UV) Licht auf das Ansprech- und Abklingverhalten des Diamantsensors. UV-Beleuchtung ist deshalb eine wichtige Alternative zu Sensorbetrieb bei erhöhten Temperaturen.

Fig. 4 zeigt, wie die Desorption von NH₃ durch Zugabe von Ozon beschleunigt wird. Man sieht in dieser Figur sehr schön, wie sich der Bauteilwiderstand am Anfang des Versuches durch Beimischung von NH₃ erhöht. Nach dem zugeführten Gaspuls setzt eine Desorption des Ammoniakgases von der Diamantoberfläche ein. Der Widerstand des Bauteiles verringert sich langsam. Führt man nun eine sehr hohe Konzentration von Ozon zu, wird der Desorptionsvorgang beschleunigt. Der Widerstand des Diamanten verringert sich während der Beigabe drastisch und bleibt auch nach der Begasung dauerhaft auf dem niedrigeren Niveau. Eine Reinigung der Sensoroberfläche ist somit mit hohen Konzentrationen von Ozon möglich.

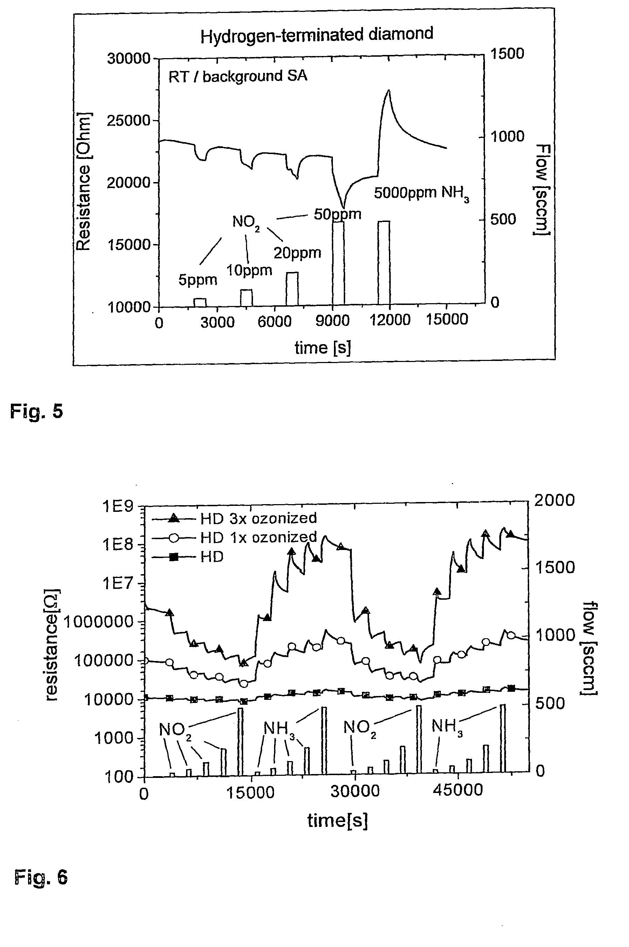

Empfindlichkeit auf oxidierende Gase:
Betrachtet man die Sensitivität einer hydrogenisierten Oberfläche auf oxidierende Gase wie z. B. Ozon und NO₂, so lässt sich - wie anhand Fig. 4 verdeutlicht - feststellen, dass bei der Begasung mit Ozon eine reinigende Wirkung an der Oberfläche einsetzt. Bei extremen Ozonkonzentrationen, die typische Umweltkonzentrationen bei weitem übersteigen, kann die Wasserstoffterminierung der Oberfläche beschädigt, bzw. partiell durch eine Sauerstoffbelegung ersetzt werden. Dieser letztere Fall wird speziell in Fig.6 weiter betrachtet.

Fig. 5 zeigt den Einfluss von NO₂. Wie man daraus entnehmen kann, verursacht NO₂ als einziges Gas eine Reduzierung des Bauteilwiderstandes bei Raumtemperatur. Demnach kann man dieses Bauteil auch zur Detektion von NO₂, und auch von weiteren Stickoxiden (NO, N20), einsetzen.

Fig.6 zeigt eine Wiederholung des in Fig. 5 gezeigten Experiments. In diesem Experiment wurde die Gasbeaufschlagungssequenz der Fig. 5 viermal wiederholt, um die Reproduzierbarkeit der Ergebnisse zu testen. Dieses Experiment wurde zunächst mit einen komplett hydrogenisierten Diamanten durchgeführt und danach zweimal wiederholt, wobei vor jeder Wiederholung der Diamant einer extrem aggressiven Sauerstoffatmosphäre ausgesetzt wurde. Bei diesen Behandlungen wird jedes Mal die Anzahl der H - terminierten Oberflächenbindungen reduziert Man erkennt dass durch die "Verdünnung" der H-Terminierung und der damit einhergehenden Erhöhung des Grundwiderstandes eine deutliche Erhöhung der Gasempfindlichkeit verursacht wird. Ein Auftreten neuer, bisher unbeschriebener Gasempfindlichkeiten wurde nach diesen Behandlungen nicht beobachtet.

Anwendungen zur Detektion von anderen Gruppe III-H-Verbindungen oder Gruppe V-H-Verbindungen:
In weiteren Experimenten, die ähnlich wie die zuvor erläuterten Experimente durchgeführt wurden, konnte nachgewiesen werden, dass mit einer hydrogenisierten Halbleiteroberfläche, insbesondere der zuvor diskutierten sensitiven Oberfläche, ebenfalls Diboran (B₂H₆) als Gruppe III-Wasserstoffverbindung sowie Phosphin (PH₃) und Arsin (AsH₃) neben NH₃ als weitere Gruppe V-Wasserstoffverbindungen detektiert werden können.

## Patentansprüche

1. Verwendung eines Substrats mit einer Wasserstoff(H)-terminierten Oberfläche zur selektiven Detektion wenigstens eines der chemischen Dotierstoffe B₂H₆, PH₃, AsH₃ in einem Gas, wobei das Substrat aus einem Nichtleitermaterial mit einer vorhandenen Oberflächenleitfähigkeit oder aus einem Halbleitermaterial gefertigt ist.

2. Verwendung nach Anspruch 1, **dadurch gekennzeichnet, dass** die Dichte der Wasserstoffbelegung an der Oberfläche durch partielle Oxidation reduziert wurde.

3. Verwendung nach Anspruch 1 oder 2, **dadurch gekennzeichnet, dass** an der Wasserstoff(H)-terminierten Oberfläche mittels einer Messeinrichtung Verschiebungen von Oberflächen ladungen gemessen werden.

4. Verwendung nach einem der vorhergehenden Ansprüche, **dadurch gekennzeichnet, dass** eine auf das Substrat aufgebrachte Schicht von Wasserstoffatomen die Wasserstoff(H)-terminierte Oberfläche bildet.

5. Verwendung nach Anspruch 4, **dadurch gekennzeichnet, dass** die Schicht von Wasserstoffatomen eine monoatomare Schicht ist.

6. Verwendung nach einem der vorhergehenden Ansprüche, **dadurch gekennzeichnet, dass** das Substrat aus Diamant, amorphem Silizium, Siliziumcarbid, einem Gruppe III-Nitrid oder einem Metalloxid gefertigt ist.

7. Verwendung nach Anspruch 6, **dadurch gekennzeichnet, dass** das Substrat GaN oder Zinnoxid oder Zinkoxid umfasst.

8. Verwendung nach einem der vorhergehenden Ansprüche, **dadurch gekennzeichnet, dass** der elektrische Widerstand an der Wasserstoff(H)-terminierten Oberfläche mittels einer Messeinrichtung gemessen wird.

9. Verwendung nach einem der vorhergehenden Ansprüche, **dadurch gekennzeichnet, dass** die Messung zur Detektion der genannten Gase oder Dämpfe bei einer Temperatur unter 100°C, insbesondere unter ca. 55°C, erfolgt.

10. Verwendung nach Anspruch 9, **dadurch gekennzeichnet, dass** die Messung bei Raumtemperatur erfolgt.

11. Verwendung nach einem der voranstehenden Ansprüche, **dadurch gekennzeichnet, dass** die sensitive Wasserstoff(H)-terminierte Substratoberfläche durch Spülung mit einem Fluid, das die zu detektierenden Stoffe nicht enthält, insbesondere Luft, gereinigt wird.

12. Verwendung nach Anspruch 11, **dadurch gekennzeichnet, dass** die Reinigung unter Zugabe eines oxidierenden Fluids, wie insbesondere Ozon, und/oder unter Zugabe von ultraviolettem Licht erfolgt.

## Claims

1. Use of a substrate having a hydrogen (H) -terminated surface for selective detection of at least one of the chemical dopants B₂H₆, PH₃, AsH₃ in a gas, wherein the substrate is fabricated from a non-conductor material having an existing surface conductivity or from a semiconductor material.

2. Use according to Claim 1, **characterized in that** the density of the hydrogen coverage on the surface was reduced by partial oxidation.

3. Use according to Claim 1 or 2, **characterized in that** the hydrogen(H)-terminated surface is measured for displacements of surface charges by means of a measuring device.

4. Use according to any one of the preceding claims, **characterized in that** the hydrogen(H)-terminated surface is formed by a layer of hydrogen atoms applied to the substrate.

5. Use according to Claim 4, **characterized in that** the layer of hydrogen atoms is a monoatomic layer.

6. Use according to any one of the preceding claims, **characterized in that** the substrate is fabricated from diamond, amorphous silicon, silicon carbide, a group III-nitride or a metal oxide.

7. Use according to Claim 6, **characterized in that** the substrate comprises GaN or tin oxide or zinc oxide.

8. Use according to any one of the preceding claims, **characterized in that** the electrical resistance on the hydrogen(H)-terminated surface is measured by means of a measuring device.

9. Use according to any one of the preceding claims, **characterized in that** the measurement for detecting the gases or vapours mentioned takes place at a temperature below 100°C, especially below approx. 55°C.

10. Use according to Claim 9, **characterized in that** the measurement takes place at room temperature.

11. Use according to any one of the preceding claims, **characterized in that** the sensitive hydrogen(H)-terminated substrate surface is cleaned by purging with a fluid that does not contain the substances to be detected, especially air.

12. Use according to Claim 11, **characterized in that** the cleaning is effected with addition of an oxidizing fluid, especially ozone, and/or with addition of ultraviolet light.

## Revendications

1. Utilisation d'un substrat muni d'une surface à terminaison hydrogène (H) pour la détection sélective d'au moins un des dopants chimiques B₂H₆, PH₃, AsH₃ dans un gaz, le substrat étant fabriqué en un matériau non conducteur ayant une conductivité de surface existante ou en un matériau semi-conducteur.

2. Utilisation selon la revendication 1, **caractérisée en ce que** la densité du revêtement hydrogène sur la surface a été réduite par oxydation partielle.

3. Utilisation selon la revendication 1 ou 2, **caractérisée en ce que** des déplacements de charges de surface sont mesurés au moyen d'un dispositif de mesure sur la surface à terminaison hydrogène (H).

4. Utilisation selon l'une quelconque des revendications précédentes, **caractérisée en ce qu'**une couche d'atomes d'hydrogène appliquée sur le substrat forme la surface à terminaison hydrogène (H).

5. Utilisation selon la revendication 4, **caractérisée en ce que** la couche d'atomes d'hydrogène est une couche monoatomique.

6. Utilisation selon l'une quelconque des revendications précédentes, **caractérisée en ce que** le substrat est fabriqué en diamant, en silicium amorphe, en carbure de silicium, en un nitrure du groupe III ou en un oxyde métallique.

7. Utilisation selon la revendication 6, **caractérisée en ce que** le substrat comprend du GaN ou de l'oxyde d'étain ou de l'oxyde de zinc.

8. Utilisation selon l'une quelconque des revendications précédentes, **caractérisée en ce que** la résistance électrique est mesurée au moyen d'un dispositif de mesure sur la surface à terminaison hydrogène (H).

9. Utilisation selon l'une quelconque des revendications précédentes, **caractérisée en ce que** la mesure pour la détection des gaz ou vapeurs mentionnés a lieu à une température inférieure à 100 °C, notamment inférieure à environ 55 °C.

10. Utilisation selon la revendication 9, **caractérisée en ce que** la mesure a lieu à température ambiante.

11. Utilisation selon l'une quelconque des revendications précédentes, **caractérisée en ce que** la surface de substrat à terminaison hydrogène (H) sensible est nettoyée par rinçage avec un fluide qui ne contient pas les substances à détecter, notamment de l'air.

12. Utilisation selon la revendication 11, **caractérisée en ce que** le nettoyage a lieu avec ajout d'un fluide oxydant, tel que notamment de l'ozone, et/ou avec ajout de lumière ultraviolette.
